# EUROPEAN PATENT APPLICATION

(11) **EP 1 394 550 A1**
(43) Date of publication of application: **03.03.2004**
(21) Application number: 02078560.6
(22) Date of filing: 30.08.2002
(51) Int. Cl.: G01N 33/68, G01N 33/569

(54) **A method of analysing relative epitope appearance in a plurality of samples**

(71) Applicant: Plant Research International B.V., 6708 PB Wageningen (NL)
(72) Inventor: Bergervoet, Johannes Hendrikus Wilhelmus, 6708 PB Wageningen (NL)
(74) Representative: De Hoop, Eric

(57) **Abstract**

The invention provides a method and materials for monitoring and isolating differentially expressed epitopes. In accordance with the method of the invention, differently labelled populations of epitopes from sources to be compared are competitively bound with a display library to provide a differential expression library which, in the preferred embodiment, may be manipulated by fluorescence-activated cell sorting (FACS). Monitoring the relative signal intensity of the different fluorescent labels on the reference display library permits quantitative analysis of expression levels relative to the reference sample. The invention also provides a method for identifying and isolating rare epitopes etc. Populations of display library having relative signal intensities of interest can be isolated by FACS and the attached epitopes etc. can be identified by two dimensional electrophoresis and, or MS-MS sequencing. Selected organisms can be isolated by FACS and cultivated or used as starting material for differential protein screening.

## Description

### FIELD OF THE INVENTION

The invention relates generally to methods for isolation of epitopes. In this specification an epitope is intended to mean a unique molecular shape or sequence carried on a metabolite, hormone, protein, protein complexes, viruses, bacteria, bacterial spores, fungi, fungal spores, yeast, algae, organelles, cells or other cellular components that bind a specific antibody or peptide.

### BACKGROUND

Generally in proteomics approaches, samples are compared after 2-D electrophoresis and the spots of interest are sequenced. Although this is a valid method by which large numbers of proteins are compared it can be optimised because the majority of the spots will be present in both samples and the method of comparison is time consuming, especially when the number of protein spots is high [1]. This will make gel evaluation rather complex. Spots derived from highly abundant proteins cause another problem. These spots can change the overall 2-D pattern and mask the presence of proteins appearing at a low level as well. Therefore a differential screening method, which eliminates proteins present in both samples, will reduce the number of proteins to be evaluated and/or sequenced.

Such a differential screening method is also desirable for species other than proteins, e.g. protein complexes, viruses, bacteria, bacterial spores, fungi, fungal spores, yeast, algae, organelles or cells that binds a specific antibody or peptide. A protein complex can be defined as a structure of one peptide or protein with one or more other proteins, peptides or other molecules. These species, including proteins, are indicated herein as epitopes.

Selection of specific markers for fungal spores is also hampered by the abundant presence of simular epitopes between related species. This will result in high cross reactivity between the species and therefore it is difficult to find, and isolate the specific markers. Using a differential screening method would make it possible to select the marker in the presence of the related (cross reactive) species.

### SUMMARY OF THE INVENTION

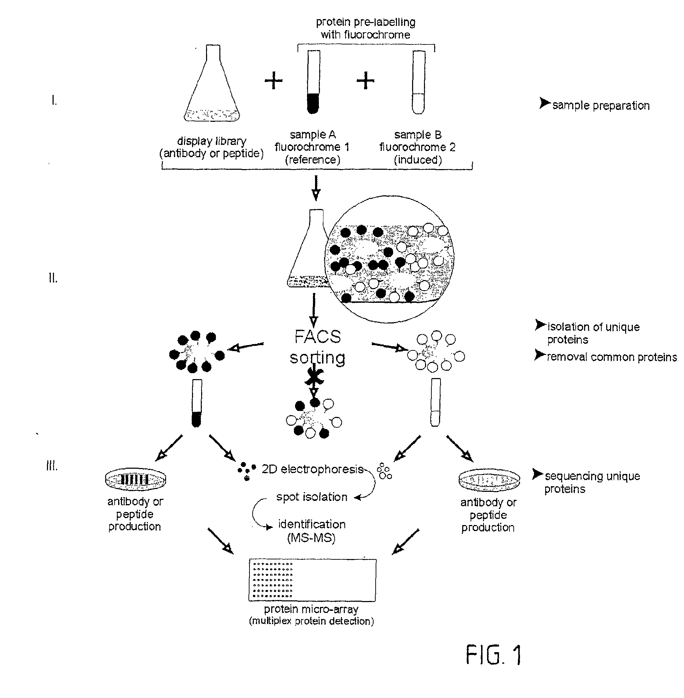

The invention comprises a method for differential protein expression analysis using a multivalent display library. In this library, the cells or particles expose multiple antibodies or peptides on their surface (one type per cell). Unique proteins are selected while proteins appearing in both samples can easily be removed (Fig. 1). Pre-stained samples, with different fluorescent probes, will bind to the display library cells and can be selected and sorted using a flow sorter. The flow sorter is used to separate the specified cells from the cells with both colours. Thus, a subtractive library is made. The pre-stained samples can, after dissociation from the display library cells, be used for identification using 2D electrophoresis. Because the proteins of the two samples are stained, both samples can be run on the same gel and due to the different fluorescent properties of both samples comparison is relatively easy. The proteins may be identified using chromatography, or other standard analytical methods like GC, LC or GCMS.

A second advantage of this technique is that it simultaneously leads to a pre-selection of the antibody or peptide repertoire of the display library. Isolated display library cells with specific antibodies or peptides recognising identified proteins can be sub-cultured. This will shorten the selection time of specific antibodies or peptides for a range of applications (e.g. protein micro-arrays, functional studies, immunoassays etc.). In addition, low abundant proteins can be isolated in this way because their relative concentration increases.
Accordingly, objects of the invention include, but are not limited to, providing a method for identifying and isolating differentially expressed proteins; providing a method of identifying and isolating proteins on the basis of labels that generate different optical signals; providing a method for profiling protein expression of large numbers of proteins; providing a method of identifying and separating proteins in accordance with whether their expression is increased or decreased under any given conditions.

More generally, it is an object of the invention to provide a method for identifying and isolating different eptitopes, and to provide a method for identifying and isolating epitopes on the basis of labels that generate different optical signals.

The present invention accomplishes these and other objects by providing a method of analysing relative epitope appearance in a plurality of different cells, tissues, organisms or a solution, the method comprising the steps of:
- providing a display library,
- providing a population of epitopes from each of the plurality of different cells, tissues, organisms or solution, and conjugating the epitopes from the different cells, tissues, organisms or solution with a different light-generating label,
- providing circumstances where the labelled epitopes can interact competitively with the display library to form multiplexes, and
- analysing and sorting the multiplexes according to a relative optical signal generated by the light-generating labels of the multiplexes.

In comparing epitope appearance, epitope populations are competitively binding display library cells, after such competitive binding a differential library is formed which may be manipulated by fluorescence-activated cell sorting (FACS), or other sorting means responsive to optical signals generated by labelled epitopes bound to the display library cells.

Monitoring the relative signal intensity of the different labels on the display library permits quantification of the relative appearance of particular epitopes in the different populations.

FACS uses electro-optical techniques to provide quantitative analysis of a variety of cellular properties, which are sequentially studied in a continuous-flow system. Cells or particles will pass one after the other and on the basis of these measured properties, the cells may then be physically isolated for use in various biological studies. Cell sorting provides the possibility to analyse and sort cells by differences in physiology, metabolism, morphology and other characteristics at rates up to thousands per second.

In one aspect of the invention, populations of display library cells having relative signal intensities of interest are isolated by FACS and the attached epitopes are identified to determine the identities of the differentially occurring epitopes.

Epitopes of major interest are proteins, and the method of the present invention is especially suitable as a differential screening method for differentially expressed proteins.

Hereinafter the invention will be specifically illustrated for proteins, it being evident that instead of proteins other epitopes may be employed as well.

The present invention overcomes shortcoming in the art by providing a means for separating and identifying proteins that are differentially expressed without requiring any previous analysis or knowledge of the identity. The invention also permits differentially regulated proteins to be separated from unregulated proteins for analysis, thereby eliminating the need to analyse large numbers of unregulated proteins in order to obtain information on the proteins of interest.

Other epitopes, like protein complexes, viruses, bacteria, bacterial spores, fungi, fungal spores, yeast, algae, organelles, can be used in a similar way. The samples to be compared are differently stained (e.g. green- or red fluorescent) and mixed with a display library. After sufficient incubation time this mixture is sorted and the display cells with only one label type captured are isolated.

The invention can be used for the isolation and identification of differentially expressed epitopes in plant cell and in the isolation and identification of tumor cells, tumor inducing agents, toxicology studies and diagnostics. Furthermore, microbe/pathogen detection and identification and microbe/pathogen host (e.g. human, animal or plant) interaction can be studied and revealed.

### BRIEF DESCRIPTION OF THE DRAWINGS

Figure 1. Overview of the method of the invention.

I. Fluorescent labels with different emission spectra are added to the samples A and B. The pre-stained samples are mixed and added to induced display library cells.
II. During incubation of this mixture, these samples will compete for the available binding places on the surface of the display library cells. When the fluorescent sample component is unique the display library cell will have a single fluorescent colour but when this component is present in both samples the display library cell will have both fluorescent colours.
III. These colour differences are used to select the unique sample display library combinations for further investigation and identification. The display library cells that show both colours are removed while the specific stained cells are collected.

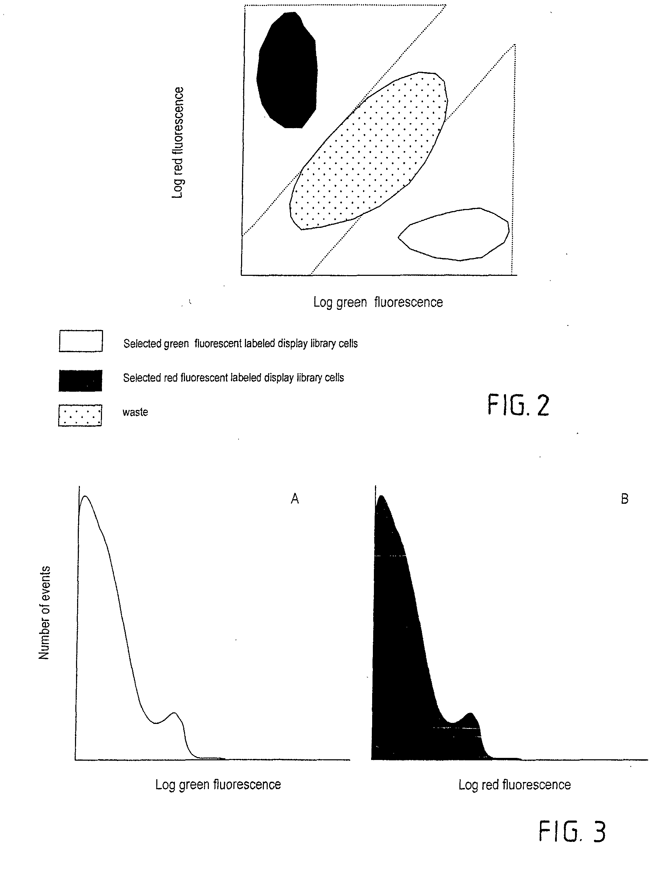

Figures 2 and 3 illustrate the theoretical FACS analysis of display library cells loaded with competitively bound proteins labelled with two different fluorescent dyes. The majority of display library cells is a 45° angle because these cells have bound fluorescent labels in a ratio of approx. 1:1. Display cells with which only one type of fluorochrome is bound, will be in clusters to the left or right (Fig. 2). When shown as one dimensional plots these cell will be at the right hand side of the cell population on the x-axis (Fig. 3).

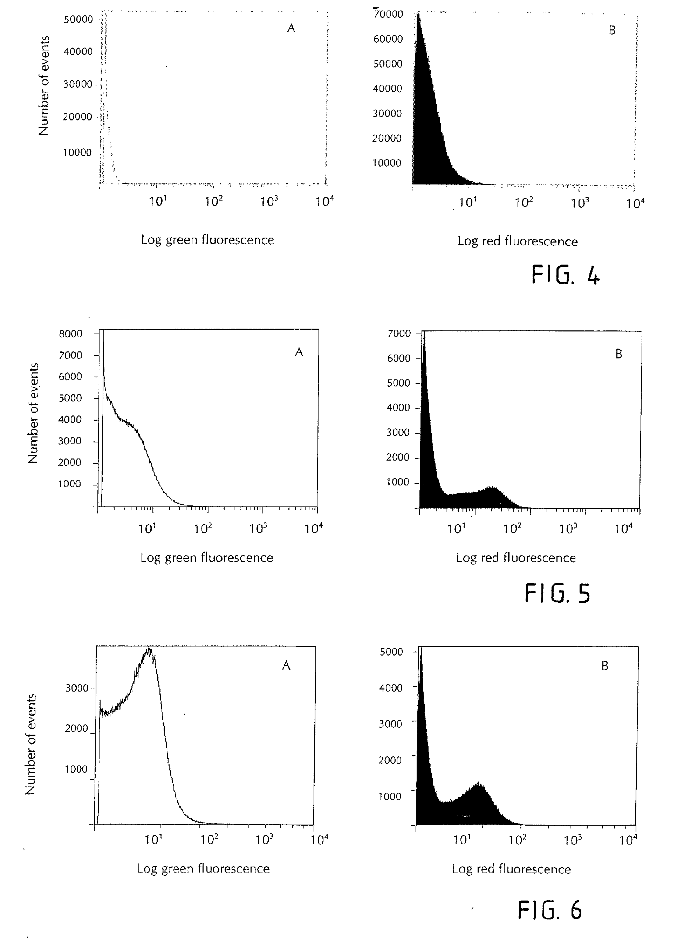

Figures 4 to 6 show one-dimensional plots of the amount of fluorescence vs. the number of events of Examples 1 to 3, respectively.

### DETAILED DESCRIPTION OF THE INVENTION

The present invention provides a means for analysing relative gene expression in a plurality of cells and/or tissues that are of interest. The plurality usually comprises a pair of cell or tissue types, such as a immature seeds or other plant material and mature seeds or other plant material, or such as a cell or tissue type being subjected to a stimulus or stress, e.g. a change of nutrients, temperature, or the like, and the corresponding cell or tissue type in an unstressed or unstimulated state. The plurality may also include more than two cell or tissue types, such as would be required in a comparison of expression patterns of the same cell or tissue over time, e.g. seeds after exposure of an organism to a candidate chemical, antagonistic microorganism, tissues, organs (liver, kidney, intestine, flowers) and the like. Preferably, the plurality is 2 or 3 cell or tissue types. For analysis in accordance with the invention, proteins are extracted from the cells or tissues of interest using conventional protocols.

An important feature of the invention is that the proteins whose expression levels change or are different than those of the other cells or tissues being examined may be analysed separately from those that are not regulated or otherwise altered in response to whatever stress or condition is being studied. As described below, in the preferred embodiment proteins from the cells or tissues of interest are competitively binding with a display library consisting of antibodies or peptides (Fig. 1, Part I). The display library carrying labelled proteins in ratios indicating differential expression may be manipulated and analysed separately from those carrying labelled proteins in ratios indicating no change in expression, e.g. "house-keeping" proteins or structural proteins.

Labelled proteins from the cells or tissues to be compared are competitively binding to the reference display library (Fig. 1, Part II). After binding, an optical signals is generated by each of the labelled species of proteins so that a relative optical signal is determined for each display library cell. Preferably, such optical signals are generated and measured in a fluorescence activated cell sorter, or like instrument, which permits the display library cell to be sorted and accumulated whose relative optical signal fall within a predetermined range of values (Fig. 1, Part III). The display library cell loaded proteins generating relative optical signals in the desired range may be isolated and identified by sequencing, such as with MS-MS.

An important feature of the invention is the use of display library cell tags which display antibodies or peptide sequences with preferably a maximum variety. Phage, E. coli and yeast display describes a selection technique in which a peptide or protein is expressed as a fusion with a coat protein of the cell, resulting in display of the fused protein on the exterior surface, while the DNA encoding the fusion resides within the cell. These displays cells have been used to create a physical linkage between a vast library of random peptide sequences to the DNA encoding each sequence, allowing rapid identification of peptide ligands for a variety of target molecules (antibodies, enzymes, cell-surface receptors, etc.) by an *in vitro* selection process called panning. In its simplest form, panning is carried out by incubating a library of displayed peptides with a plate (or bead) coated with the target, washing away the unbound cells, and eluting the specifically-bound cells. The eluted cell is then amplified and taken through additional cycles of panning and amplification to successively enrich the pool of cells in favour of the tightest binding sequences. After 3-4 rounds, individual clones are characterised by DNA sequencing and ELISA [2-4].

An important aspect of the invention is the sorting and attachment of labelled proteins to display library cells or to separate regions on a solid phase support such that each display library cell or region has substantially only one kind of protein attached. This objective is accomplished by insuring that substantially all different display library cell have different antibodies or peptides expressed.

After the display libraries are prepared for specific binding, the pre-stained proteins are mixed with display library cells under conditions that favour the formation of perfectly binding between the proteins and the display library cells. Finally, the display library cells are washed to remove proteins that have not bound.

Specificity of the binding of proteins to their complements may be increased by taking a sufficiently small sample so that both a high percentages of proteins in the sample are unique and the nearest neighbours are substantially different. Preferably, loaded display library cells may be separated from unloaded display library cells by a fluorescence-activated cell sorting (FACS) instrument using conventional protocols.

### Competitive binding and Light-Generating Labels

Proteins from the cells and/or tissues being analysed are isolated and labelled by conventional techniques (vide e.g. [5]). Preferably, a light-generating label is conjugated to the proteins providing a fluorescent label for identification. A large number of light-generating labels are available, including fluorescent, calorimetric, chemiluminescent, and electroluminescent labels. Generally, such labels produce an optical signal which may comprise an absorption frequency, an emission frequency, an intensity, a signal lifetime, or a combination of such characteristics. Preferably, fluorescent labels are employed, either by direct conjugation of fluorescently labelled fluorchromes or by indirect application by incorporation of a capture moiety, such as biotinylation, followed by complexing with a moiety capable of generating a fluorescent signal, such as a streptavidin-fluorescent dye conjugate or a fluorescent label. Preferably, the optical signal detected from a fluorescent label is an intensity at one or more characteristic emission frequencies. Selection of fluorescent dyes and means for attaching or conjugating them to proteins is well known (vide e.g. [5]).

Preferably, light-generating labels are selected so that their respective optical signals can be related to the quantity of labelled proteins present and so that the optical signals generated by different light-generating labels can be compared. Measurement of the emission intensities of fluorescent labels is the preferred means of meeting this design objective. For a given selection of fluorescent dyes, relating their emission intensities to the respective quantities of protein strands requires consideration of several factors, including fluorescent emission maxima of the different dyes, quantum yields, emission bandwidths, absorption maxima, absorption bandwidths, nature of excitation light source(s), and the like. Guidance for making fluorescent intensity measurements and for relating them to quantities of analytes is available in the literature relating to chemical and molecular analysis, e.g. [6-8]. As used herein, the term "relative optical signal" means a ratio of signals from different light-generating labels that can be related to a ratio of differentlly labelled proteins. Preferably, a relative optical signal is a ratio of fluorescence intensities of two or more different fluorescent dyes.

Competitive binding between the labelled protein derived from the plurality of cells or tissues is carried out by applying equal quantities of the labelled protein from each such source to the display library. The particular amounts of labelled proteins added to the competitive binding vary widely depending on the embodiment of the invention. Factors influencing the selection of such amounts include the quantity of display library cells used, the type of display library used, the complexity of the populations of labelled proteins, and the like. The competitive binding conditions are selected so that the proportion of labelled proteins forming complexes is directly proportional to the amount of that protein in its population in comparison with the amount of the competing proteins of identical sequence in their respective populations.

### Flow sorting of display library cells with Up-Regulated and/or Down-Regulated Gene Products

Display library cells containing fluorescently labelled epitopes are conveniently classified and sorted by a commercially available FACS instrument, e.g. [9]. Preferably, upon excitation with one or more high intensity light sources, such as a laser, a mercury arc lamp, or the like, each display library cell will generate fluorescent signals, usually fluorescence intensities, related to the quantity of labelled epitopes from each cell or tissue types carried by the display library cell. As shown in FIG. 2, when fluorescent intensities of each display library cell are plotted on a two-dimensional graph, display library cells indicating equal expression levels will be on or near the diagonal of the graph. Up-regulated and down-regulated epitopes will appear in the off-diagonal regions (Fig. 2). Such display library cells are readily sorted by commercial FACS instruments by graphically defining sorting parameters to enclose one or both off-diagonal regions as shown in FIG. 2.
One dimensional representation of this data will show the display library cells indicating equal expression levels near the y-axis and the up- and down-regulated epitopes will appear more the right hand side; for both fluorochromes separate plots have to be made (Figure 3A and B). Only selected cells are used. Display library cells with both epitope-fluorescent are not selected and therefore not shown in the histogram.

### Identification of Sorted proteins by mass spectrometry

Mass spectrometers are an analytical tool used for measuring the molecular weight (MW) of a sample. For large samples such as biomolecules, molecular weights can be measured to within an accuracy of 0.01% of the total molecular weight of the sample i.e. within a 4 Daltons (Da) or atomic mass units (amu) error for a sample of 40,000 Da. This is sufficient to allow minor mass changes to be detected, e.g. the substitution of one amino acid for another, or a post-translational modification.
For small organic molecules the molecular weight can be measured to within an accuracy of 5 ppm, which is often sufficient to confirm the molecular formula of a compound, and is also a standard requirement for publication in a chemical journal.
Structural information can be generated using certain types of mass spectrometers, usually tandem mass spectrometers, and this is achieved by fragmenting the sample and analysing the products generated. This procedure is useful for the structural elucidation of organic compounds, for peptide or oligonucleotide sequencing, and for monitoring the existence of previously characterised compounds in complex mixtures with a high specificity by defining both the molecular weight and a diagnostic fragment of the molecule simultaneously e.g. for the detection of specific drug metabolites in biological matrices.

### Identication of epitopes by conventional analytical methods

The isolated epitopes can be identified using conventional methods such as TLC, GC, HPLC, FPLC, LC, GCMS and other methods of choice.

### Identification of sorted proteins by conventional 2D

After selection of the specific cells by flow sorting both samples are concentrated and the proteins are isolated. These protein samples are then:
- separated according their iso-electric point using standard iso-electric focusing procedures
- separated according their molecular weight using standard SDS-PAGE procedures
- since the proteins are prestained and their fluorescent label is unique for the sample they can directly be visualized using a laser scanner, the proteins of the display cells are not fluorescently stained and therefore not visible.

Because pre-staining can change iso-electric point and/or molecular weight, care has to be taken when comparing the protein spots but these spots are unique and therefore ready to use for MS-MS identification.

### Examples

### Example 1

Proteins were isolated from high quality seed and germinated high quality seed using Leamlli buffer. The proteins were precipitated using ice-cold (-20 °C) TCA aceton and centrifugated at 14000g for 10 minutes at 4 °C. The pellet was washed twice in ice-cold aceton and air-dried before resuspending in PBS buffer including a proteinase inhibitor coctail (Complete™ , Roche Diagnostics) . After adjusting the protein concentration the fluorochrome conjugation was performed according the manufacturers protocol (Molecular Probes, Eugene, Oregon, USA). The two samples were mixed and added to a non-induced or an induced yeast display library. The non-induced yeast cell displayed no peptides on their cell walls whereas the induced yeast cell displayed constrained and non-constrained peptides. The total variation of these peptides was approx. 10⁶. The pre-stained protein samples plus display mixture were incubated for one hour at room temperature before sorting.

The laser alignment, dual laser configuration and the sorter were checked and set-up using standard fluorescent beads. In the one-dimensional plots the x-axis represents the amount of fluorescence and the y-axis represents the number of events recorded during approx. 30 seconds. The actual sort took a longer time.

Fluorescent pre-stained protein samples (labelled with a green (A) or red (B) fluorescent label) were added to a display library without exposure or expression of peptides or antibodies at the cell walls. The suspension of non-induced yeast cells and fluorescently pre-stained proteins were measured (FACS) and no fluorescent display library cells were detected and sorted, confirming that no a-specific binding occurred. The results are shown in Fig. 4.

### Example 2

When pre-stained protein samples (according example 1) were added to an induced display library for the first time (with exposure or expression of peptides or antibodies at the cell walls), yeast cells with a fluorescent level higher as 10¹ were isolated. Fluorescent prestained proteins bind to display library cells and these cells could be detected and sorted using fluorescent activated cell sorting (FACS). The isolated yeast cells were transferred to fresh medium and cultured. The results are shown in Fig. 5.

### Example 3

Fluorescent pre-stained protein samples (according example 1) were added to the enriched induced yeast cells of example 2.

Pre-labelled samples bind to these display library cells and show an enrichment for the labelled proteins. These cells can be detected and sorted using fluorescent activated cell sorting (FACS) .Cells with a fluorescent level higher as 10¹ were isolated. The isolated cells were transferred to fresh medium and cultured. The results are shown in Fig. 6.

### Example 4

Isolated fluorescently labelled proteins of example 3 are dissociated from the display library cells and directly used for 2D electrophoresis using conventional techniques. Since the proteins are pre-labelled gel evaluation is less complex. The unique spots are then isolated and used for identification by MS-MS.

### Example 5

From the set of isolated or identified epitopes the best ones can be used to pickup their display counterparts. These display cells can be used directly or indirectly for the preparation of arrays which can be used as tools in R&D and diagnostics.

### References

[1] Görg et all (2000. The current state of the art of two-dimensinal electrophoresis with immobilized pH gradients. Electrophoresis 21, 1037-1053.
[2] Marks et al. (1991) By-passing immunization. Human antibodies from V-gene libraries displayed on phage. J. Mol. Biol. 222, 581-97.
[3] Hoogenboom et al. (1991) Multi-subunit proteins on the surface of filamentous phage: methodologies for displaying antibody (Fab) heavy and light chains. Nucleic Acids Res. 19, 4133-7.
[4] Kanppik A. et al. (2000). Fully synthetic human combinatorial antibody libraries (HuCAL) based on modular consensus frameworks and CDRs randomized with trinucleotides. J. Mol. Biol., 296, 57-86.
[5] Haugland R.P. (2002) Handbook of fluorescent probes and research products. Molecular Probes, Eugene, OR, USA.
[6] Guilbault G.C. (Editor), Practical Fluorescence Second edition, (Marcel Dekker, New York, 1990).
[7] Pesce A.J. Fluorescence spectroscopy; an introduction for biology and medicine. M. Dekker; ASIN: 082471539X
[8] White et al, Fluorescence Analysis: A practical Approach (Marcel Dekker, New York, 1970)
[9] Van Dilla et al, Flow Cytometry: Instrumentation and Data Analysis (Academic Press, New York, 1985).

## Claims

1. A method of analysing relative epitope appearance in a plurality of different cells, tissues, organisms or a solution, the method comprising the steps of:
- providing a display library,
- providing a population of epitopes from each of the plurality of different cells, tissues, organisms or solution, and conjugating the epitopes from the different cells, tissues, organisms or solution with a different light-generating label,
- providing circumstances where the labelled epitopes can interact competitively with the display library to form multiplexes, and
- analysing and sorting the multiplexes according to a relative optical signal generated by the light-generating labels of the multiplexes.

2. The method of claim 1 wherein the light-generating labels are fluorescent labels.

3. The method of claim 1 wherein the epitope is a protein.

4. The method of claim 1 wherein said relative optical signal is a ratio of fluorescence intensities and wherein said plurality of different cells or tissues is two.

5. A method of isolating epitopes derived from a plurality of different cells or tissues, the method comprising the steps of:
- providing a population of peptides derived from a display library; and
- each epitopes having a light-generating label capable of generating an optical signal indicative of the cells or tissues from which it is derived;
- competitively binding of the populations of epitopes in each of the plurality of different cells or tissues
- epitopes from each of the different cells or tissues such that common epitopes from the different cells or tissues bind with the same peptides or antibodies of the display library and;
- epitope populations are present in multiplex on each of the display library cells in ratios directly related to the relative appearance of their corresponding epitopes in the different cells or tissues; and
- isolating epitopes corresponding to epitopes differentially expressed in the different cells or tissues by sorting display library cells in accordance with the optical signals generated by the populations of epitopes binding thereto.

6. The method of claim 5 wherein said plurality of different cells or tissues is two and wherein said optical signal is a fluorescent signal.

7. The method of claim 6 wherein said populations of epitopes are labelled with different fluorescent labels.

8. The method of claim 7 wherein said step of isolating includes sorting said display library cells in accordance with the ratio of fluorescence intensities generated by said populations of epitopes bound thereto.

9. The method of claim 8 wherein said step of isolating includes sorting said display library cells with a fluorescence-activated cell sorter.

10. The method of claim 9 further including the step of identifying said isolated epitopes by determining the identity of each said isolated epitope.
